Europäisches Patentamt

European Patent Office

Office européen des brevets

(11) Publication number: **0 154 066**

A1

# EUROPEAN PATENT APPLICATION

(21) Application number: 84301466.3

(22) Date of filing: 06.03.84

(51) Int. Cl.⁴: **C 12 P 21/00**
C 12 P 21/02, A 61 K 35/84
A 61 K 37/00
//A61K39/39, A61K45/05

(43) Date of publication of application:
11.09.85 Bulletin 85/37

(84) Designated Contracting States:
DE FR GB

(71) Applicant: NODA SHOKKIN KOGYO CO., LTD.
121, Shimizu
Noda-shi Chiba-ken(JP)

(72) Inventor: Sugano, Nobuhiko
313-3 Nagae
Toyama-shi Toyama-ken(JP)

(72) Inventor: Choji, Yoko
2314 Tsujigadou Mizuhasi
Toyama-shi Toyama-ken(JP)

(72) Inventor: Takarada, Tetsuhito
977-4 Kawashimada
Gotenba-shi Shizuoka-ken(JP)

(72) Inventor: Maeda, Hiroaki
2355 Yamazaki
Noda-shi Chiba-ken(JP)

(74) Representative: Skailes, Humphrey John et al,
Frank B. Dehn & Co. Imperial House 15-19 Kingsway
London WC2B 6UZ(GB)

(54) Immunopotentiation agents.

(57) An immunopotentiation agent comprising two types of peptidoglycans whose peptide portions contain acidic amino acids and whose polysaccharide portions contain arabinose, glucose, galactose, mannose and xylose, the first of which peptidoglycans has a molecular weight of 500,000 to 800,000 and the second of which has a molecular weight of 10,000 to 80,000 and has a higher acidic amino acid content than the first; said agent being obtainable by culturing basidiomycetes mycelia on a xylose-rich solid medium, and extracting the mixture of mycelia and nutrient medium with water.

The extract may be treated with alcohol to precipitate the agent in purer form. The two principal peptidoglycans may be separated by chromatography.

## IMMUNOPOTENTIATION AGENTS

This invention relates to an immunopotentiation agent and more particularly an immunopotentiation agent which contains two principal peptidoglycans and is obtainable by culturing basidiomycetes mycelia.

Relationships between infectious diseases caused by bacteria, viruses and eumycetes or carcinogenesis and the immunity of the organism have been medically studied and established. Further, it has been found that a decrease of immunological competence is related to various infectious diseases and induces carcinogenesis, and that the immunological competence is further decreased by the presence of carcinoma, and sequentially affects different cells and substances in the organism, since the defence mechanism or organism is complicated.

At present, the immunological mechanism is classified into humoral immunity and cell-mediated immunity. The former shows principal functions such as antigen precipitation with immunoglobulin, phagocyte activity or the like, and protects organisms from systemic infectious diseases. The latter exhibits the principal function of activating macrophages with various lymphokins produced from T-cells, and is involved with the exclusion of cell-parasitic antigens.

Accordingly, it is apparent that infectious diseases can be prevented and treated through the prevention of a decrease of either or both of the humoral immunity and the cell-mediated immunity, and that that the immunological mechanism is weakened due to various factors such as aging, drug administration, strain and the like, and carcinogenesis is therefore induced as a result of a lowering of resistance to many infectious diseases. In view of this fact, there have been generally developed a wide variety of vaccines capable of producing antibodies having low toxicity. However, these vaccines are not satisfactory, because there are many infectious diseases caused by microorganisms which are commonly considered to be of low toxicity and there are many anitgens having no vaccines therefor. It is therefore desirable to promote general immunological enhancement. Many of the prior immunopotentiation agents which have been already proposed, however, cause severe side-effects or induce anaphylaxis. Thus they have both advantages and diadvantages.

Japanese Patent Publication 57-206618 describes the extraction of anticarcinogenic substances from basidiomycetes mycelia.

According to the present invention, there is provided an immunopotentiation agent comprising two types of peptidoglycans whose peptide portions contain acidic amino acids and whose polysaccharide portions contain arabinose, glucose, galactose, mannose and xylose, the first of which peptidoglycans has a molecular weight of 500,000 to 800,000 and the second of which has a molecular weight of 10,000 to 50,000 and has a higher acidic amino acid content than the first; said agent being obtainable by culturing basidiomycetes mycelia on a xylose-rich solid medium, and extracting the mixture of mycelia and nutrient medium with water.

The immunopotentiation agents according to the present invention have low toxicity and are effective in the prevention of a wide variety of infectious diseases.

The immunopotentiation agents of this invention have no observable side-effects. Characteristically, they can enhance the immunological competence of both the humoral immunity and cellmediated immunity. Accordingly, they can show a wide effective spectrum against viruses such as Hepatitis virus, Influenza virus, Herpes virus and the like.

It has heen found that the immunopotentiation agents of this invention have an excellent immunological enhancement effect against diseases, for example, cancer, virus diseases, immunodeficiency disease (allergic disease) and the like. Further, although the mechanism of this enhanced immunogolical competence has not yet been completely established, it is believed that it results from an increase in antibody production cells or enhancement of antibody production capability in the presence the peptidoglycans present in the agent. Furthmore, the immunopotentiation agents of this invention are also effective against a wide variety of mycotic infectious diseases, typically those caused by candida albicans. In this connection, it is noted that mycotic infectious diseases whose pathogenicity is generally low are mainly induced as a result of a lowering of immune state as a result of consumptive basal diseases such as malignant tumours, collagen diseases or other diseases; and the induction of malignant tumours is partially similar to that of mycotic infectious diseases. In conclusion, it will be understood that the immunopotentiation agents according to the present invention can be widely and effectively used in the medical field.

In the preparation of the agents of the invention, a xylose-rich culture medium should be used. Typical examples of useful media include bagasse, wheat or barley straw, rice plant straw, corn stalk and leaf, monocotyledonous plants such as sugi (i.e. Japanese cedar), or the like. A particularly useful nutrient medium is rice bran.

Further, typical examples of basidiomycetes useful in the practice of the present invention include Leninus edodes, Pleurotus ostreatus, Flammulina velutipes, Pholiota nameko, and Lyophyllum shimeji. It has been found that the anticarcinogenic substances extracted from the culture of Lentinus edodes mycelium have the most excellent activity.

The aqueous extraction of the mixture of mycelia and water is preferably performed at 40 - 60°C and at a pH of 3 - 8.

After extraction, the mixture may be filtered and sterilised. The extracted solution may then be concentrated if desired and/or dried, e.g. by freeze-drying. Preferably however the aqueous extract (or an aqueous solution or suspension prepared from dried extract) is treated with an alcohol to precipitate the immunopotentiation agent as a solid. This technique produces a purer product.

The alcohol may for example be a $c_{1-4}$ alkanol such as methanol, ethanol, propanol or butanol, preferably ethanol. About 4 volumes of the alcohol are preferably used per volume of aqueous extract.

The presence of the peptidoglycans, whose principal constituent is xylose, in the immunopotentiation agents of the invention can be confirmed by gas chromatography.

The two peptidoglycan fractions may if desired be separated, for example by chromatography, e.g. as desired below.

The present invention will be further illustrated by the following examples.

Preparation:

Solid medium composed of a blend of 90% of powered bagasse, 5% of rice bran and 5% of nutrients (wheat bran and the like) was conventionally sterilized. A solid inoculum of Lentinus endodes mycelium was then inoculated into the sterilized medium. After inoculation, the medium was transferred into an incubation room air-conditioned to a room temperature of 10 to 20°C and a relative humidity of 60%, to culture the mycelium.

After culture has been completed, the medium was transferred to a growth room, and left to stand.

The formation of fruit bodies was soon started on the surface of the medium. At this point, the medium was removed from the growth room, and broken down with a conventional disruption device to about thumb size. The disrupted medium is a mixture of the mycelium and the culture medium, and is herein referred to as a culture of mycelium.

Six hundreds grams of the disrupted medium of culture of mycelium was charged into a container, and 5 l of fresh water having a controlled pH value of from 3 to 8 was then added. Mixing and stirring of the contents of the container were made for a certain period under stepwise or gradually varied temperature conditions. Namely, during the mixing and stirring, the temperature was varied within the range of from 40°C to 60°C depending upon the pH in each of the steps. The temperature was changed to accelerate enzymation in conformity with the culture of mycelium, for example, cellulase, chitinase, glucosidase or protease. Finally, the temperature was elevated to 60°C, and mixing and stirring were made at the same temperature in order to simultaneously attain deactivation of the enzymes. As a result, mycelium ingredients, metabolic products of mycelium and ligneous decomposition products in the medium ingredients were dissolved in water.

The thus obtained suspension was placed in a filtration bag made of flannel fabrics, and then filtered under pressure. The filtrate was further filtered through a membrane filter, and then sterilized. The extracted solution or extract was obtained.

The extract obtained in the previous process was lyophilized to make powered materials. These materials are hereinafter referred to as LEM ("LEM" is an abbreviation for Lentinus edodes mycelia).

Experiment 1:

Object:

This experiment was performed to study an effect of LEM on activation of macrophages.

Laboratory:

Laboratory of Biology, Faculty of Pharmaceutical Sciences, Toyama Medical and Pharmaceutical University (Japan)

Specimens:

(i)     LEM was precipitated in alcohol, and the resulting precipitate was fractionated on a column of Sepharose 6B.  Fractions indicating two peaks were obtained.  These two fractions were designated as LAP 1 and LAP 2.

(ii)     Macrophages of guinea pig-induced peritoneal exudate cells.

(iii)     Proteins of rat-ascites hepatoma (AH 414)

Experimental method:

Donryu rats were transplanted with AH 414. Before (the day before transplantation), during and after transplantation, LAP 1 or LAP 2 was intra- peritonealy administered to these rats every day. For the first 2 to 8 days after AH 414 transplantation, spleens were removed and treated to obtain their cells.  In a glass capillary tube, the guinea pig macrophages, spleen cells and solution of antigen were put, in order, to made superposed layers, and cultured in a Petri dish for 48 hours.  After the culture has been completed, the area occupied by the migrated cells of macrophage was determined.

Results;

Activation of macrophages was confirmed for a group of LAP 1-administered Donryu rats (during the first 4 to 6 days after LAP 1 administration). However, no activation of macrophages was confirmed for a group of LAP 2-administered Donryu rats.

Analysis showed that LAP 1 used herein is peptidoglycan having a molecular weight of 500,000 to 800,000 and which mainly contain polysaccharides comprising arabinose, glucose, galactose, mannose and xylose, and produced amino acids, particularly acidic amino acids.

Analysis also showed that LAP 2 is a peptidoglycan of similar constitution but lower molecular weight (10,000 - 50,000, preferably about 30,000) and higher acidic amino acid content.

Experiment 2:

Object:

This experiment was performed to study a polyclonal activator activity of LEM.

Laboratory:

Laboratory of Biochemistry, Faculty of Medical Sciences, Osaka City University (Japan)

Experimental method:

Lymphocyte-rich mononuclear cells were isolated from normal human peripheral blood by the centrifugal method of Ficoll-Conray. The isolated cells were then added with an Eagle MEM solution containing 10% inactivated fetal calf serum to prepare a cell suspension containing $2 \times 10^6$ cells/ml.

Different concentrations of LEM were added to the cell suspension, and incubated at 37°C for 72 hours. The number of the resulting anti-TNP-SRBC antibody production cells was determined by the plaque technique.

Results:

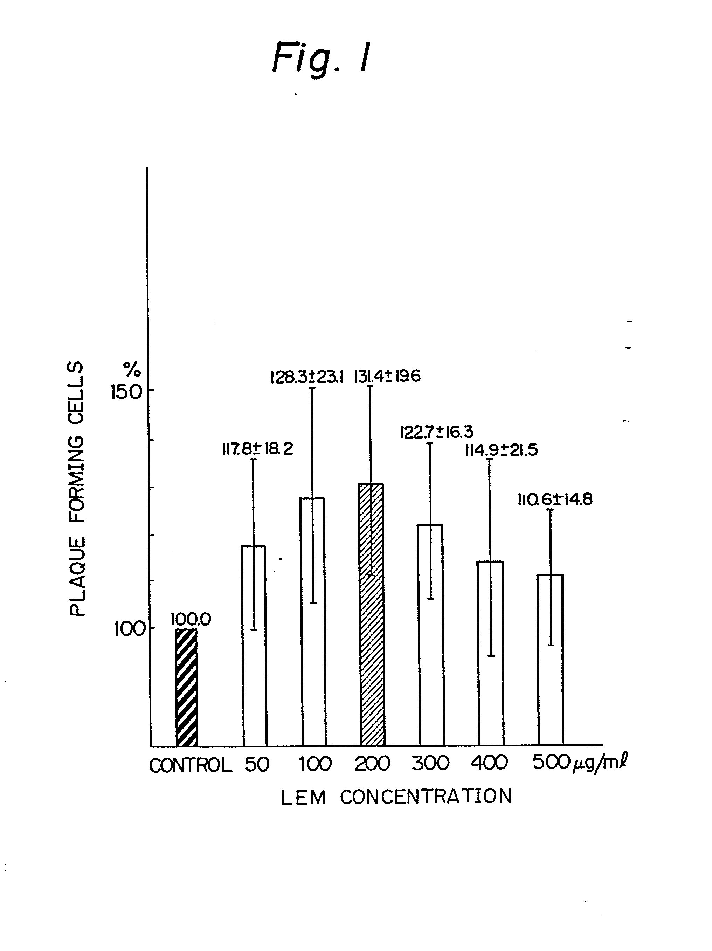

Figure 1 indicates that the addition of 100 to 200 g$^{-6}$/ml of LEM increases the number of resulting antibody production cells significantly, in comparison to the control which does not contain LEM added thereto. From this result, it is concluded that LEM possesses polyclonal activator activity, while the activity is relatively weak.

Experiment 3

Object:

This experiment was performed to study an effect of LEM on the antibody production of PWM-
stimulated mononulear cells of peripheral blood.

Laboratory:

Laboratory of Biochemistry, Faculty of Medical
Sciences, Osaka City University (Japan)

Experimental method:

A suspension of mononuclear cells containing
$2 \times 10^6$ cells/ml was prepared from normal human
peripheral blood in accordance with the manner
described in Experiment 2. 50 $m^{-6}$/ml of pokeweek
nitrogen (PWM) was then added to the suspension,
and different concentrations of LEM were simultaneously
added. The mixture was incubated at 37°C for 72
hours. The number of resulting anti-TNP-SRBC antibody
production cells was determined by the plaque technique.

Results:

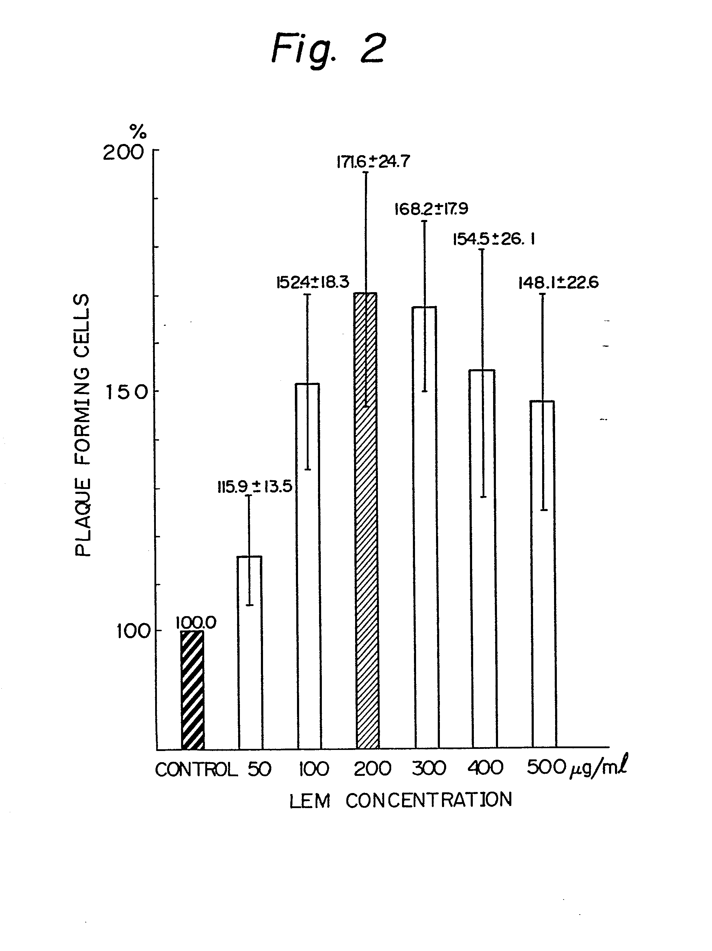

For the cell suspension to which LEM was
not added, the number of resultant anti-TNP-SRBC
antibody production cells was 632 $\pm$ 109 per 1 x
$10^6$ of viable cells. In contrast, the antibody
production for the LEM-added cell suspension was
significantly accelerated through the addition
of LEM. Results are summarized in Figure 2.

In Figure 2, the number of the anti-TNP-SRBC
antibody production cells for the LEM-added cell
suspension is indicated in percentage with regard
to 100% of the control which contains no LEM.
In the graph of Figure 2, it should be particularly
noted that the number of the anti-TNP-SRBC antibody
production cells resulted when 200 $g^{-6}$/ml of LEM
was added to the suspension is about 1.7 times
as much as that of the control. The results indicate
that LEM can promote the antibody production as
a result of its effect on either lymphocytes or
monocytes, both of which concern the antibody production.

Experiment 4
Object:

This experiment was performed to study an effect of culture supernatant of LEM-treated macrophages on lymphocyte growth.

Laboratory:

Laboratory of Biochemistry, Faculty of Medical Sciences, Osaka City University (Japan)

Experimental method:

Different concentrations of LEM were added to macrophages of guinea pig-induced peritoneal exudate cells, and cultured for 6 hours. After culture has been completed, the culture supernatant was separated from the culture medium.

On the other hand, the suspension of mononuclear cells containing $1 \times 10^6$ cells/ml was prepared from normal human peripheral blood as in Experiment 2. To 1 ml of the resulting suspension, 0.1 ml of the previously prepared culture supernatant of LEM-treated macrophages was added, and cultured for 48 hours. Thereafter, 1 uci/ml of $^3$H-thymidine (specific activity = 5 Ci/mmol) was added, and further cultured for 24 hours. After the completion of culture, lymphocyte growth was determined in the resulting culture supernatant. The determination of lymphocyte growth were made on the basis of $^3$H-thymidine uptake into acid-insoluble fractions.

Similarly, the culture supernatant of macrophages treated with different concentrations of LEM, together with PWM (described in Experiment 3), was added to the suspension of mononuclear cells of normal human peripheral blood, and cultured for 48 hours. Thereafer, $^3$H-thymidine was added, and further cultured for 24 hours. After culture has been completed, the lymphocyte growth in the supernatant was determined.

Results:

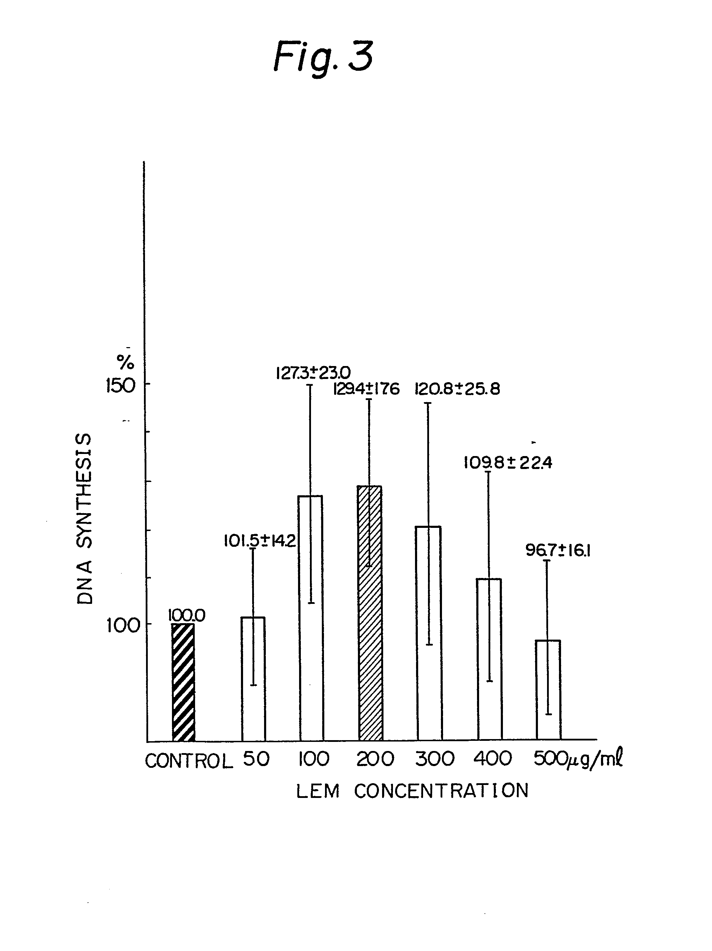

Figure 3 indicates, with regard to the cell suspension to which PWM was not added, that the culture supernatant of macrophages treated with 100 to 200 $g^{-6}$/ml of LEM has an activity and therefore growth of human mononuclear cells (i.e., DNA synthesis) is significantly accelerated. From this fact, it is believed that there is a possibility that the polyclonal antibody production of LEM which was illustrated in Figure 1 is partially affected with the activation of macrophages,.

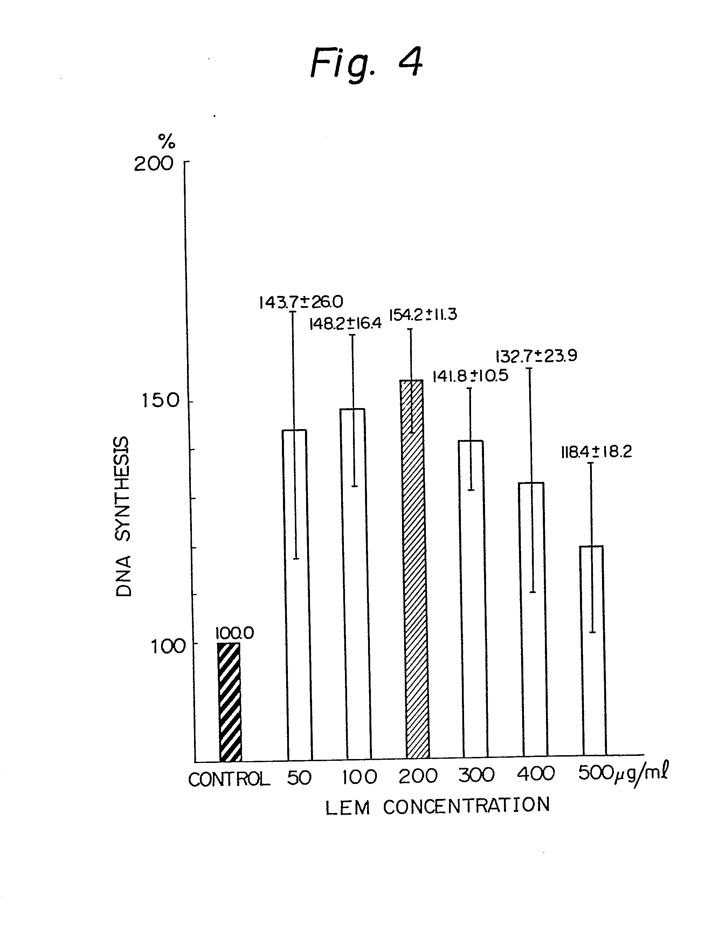

Further, Figure 4 indicates that the lymphocyte growth in the PWM-stimulated mononuclear cells, when the culture supernatant of macrophages treated with 50 to 300 $g^{-6}$/ml of LEM is added to the cell suspension, is significantly increased and is about 1.5 times as high as that of the control in which the macrophages used are not treated with LEM. It is therefore believed that the increase of the antibody production of PWM-stimulated mononuclear cells due to presence of LEM is induced by the activation of macrophages.

However, it is also possible to conceive that the lymphocytes are directly affected with LEM, since a minor amount of LEM is contained in the culture supernatant of macrophages. Assuming that LEM acts directly on the lymphocytes, it is believed that such direct action of LEM is very weak, because the amount of the culture supernatant added to the suspension of mononuclear cells was only 0.1 ml per 1 ml of the suspension.

Experiment 5:

Object:

This experiment was performed to study fractions of LEM-treated macrophage supernatant, and the effect of each of said fractions (subfraction) on lymphocyte growth.

Laboratory:

Laboratory of Biochemistry, Faculty of Medical Sciences, Osaka City University (Japan)

Experimental method:

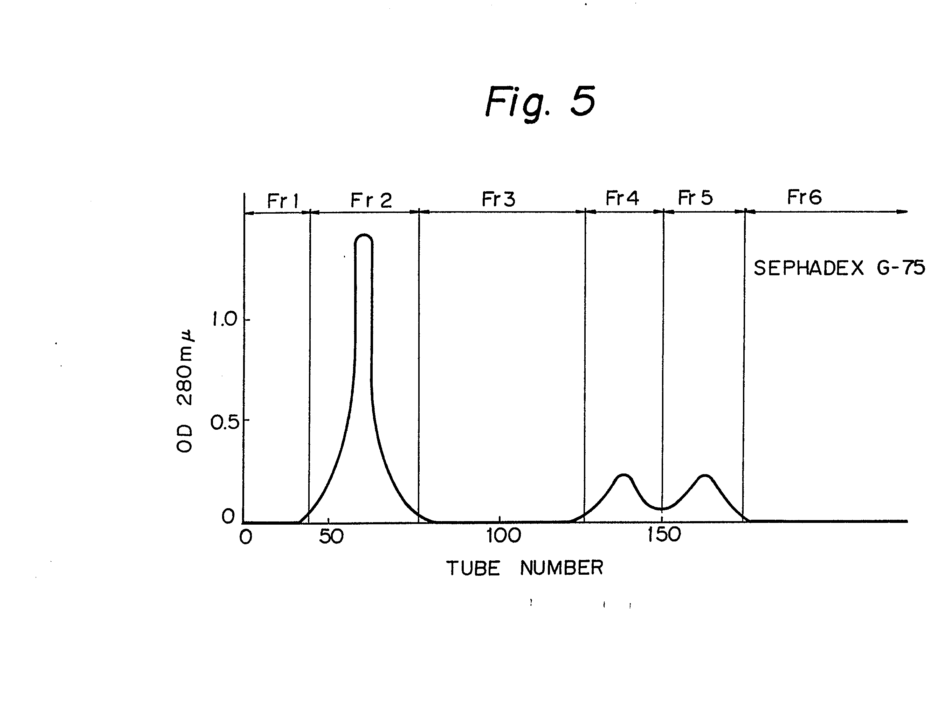

The culture supernatant of macrophages treated with LEM was prepared as in Experiment 4. The resulting LEM-treated macrophage supernatant was fractionated on a column of Sephadex G-75 (column size = 5 cm x 150 cm). The absorbance of optical density (OD) of the fractions was determined at 280 nm, and the absorbance data were plotted to obtain a curve of protein elution of the fractions. The plotted curve is shown in Figure 5.

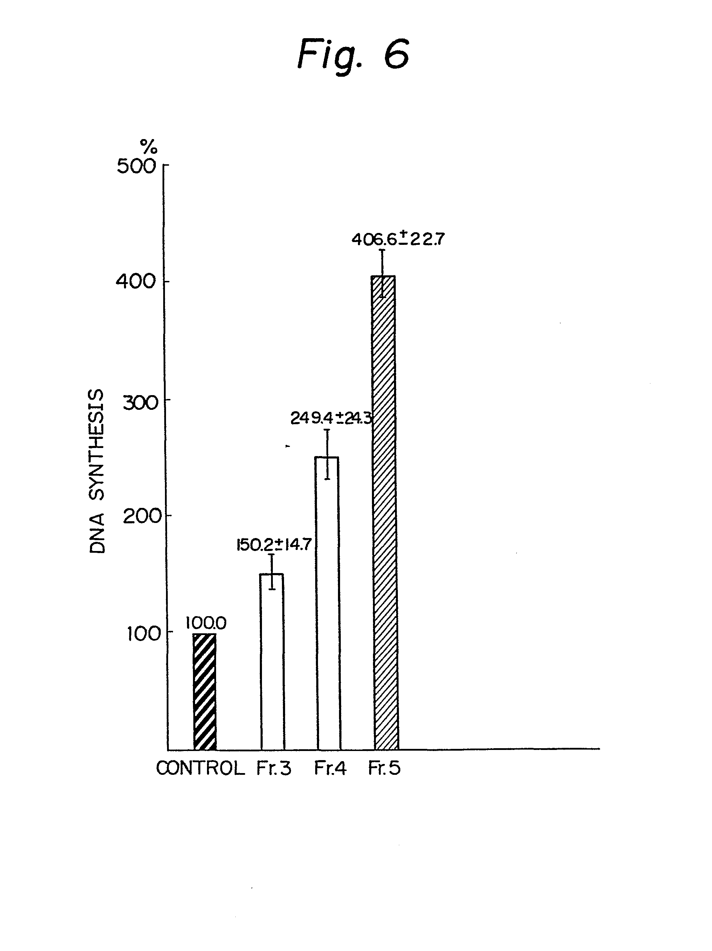

The fractions or eluates were then separated into four fractions, i.e., control, Fraction 3, Fraction 4 and Fracion 5, in conformity with the elution pattern of Figure 5. The graph of Figure 6 showing effects of subfraction of culture supernatant of LEM-treated macrophages on Con A-induced lymphoctye growth was obtained.

Each of the separated fractions was concentrated to correct its volume to the starting volume (before fractionation). 0.1 ml of each fraction, together with 20 $g^{-6}$/ml of Con A, was added to 1 ml of the suspension of mononuclear cells (2 x $10^6$ cells/ml) which was previously prepared as in Experiment 2. The addition of $^3$H-thymidine and determination of lymphocyte growth were made as in Experiment 4.

Results:

Figure 6 indicates that the Con A-induced lymphocyte growth of DNA synthesis is significantly increased through the addition of culture supernatant of LEM-treated macrophages, and an increase of DNA synthesis is typical in Fraction 5 (about 4 times of the control). Based on these results, it is believed that there is a possibility that Interleukin 1 is present in the culture supernatant of LEM-treated macrophages.

Experiment 6:
Object:

This experiment was performed to study the protective effect LEM on hepatocyte injury induced by the culture supernatant of activated macrophages.

Laboratory:

Laboratory of Biochemistry, Faculty of Medical Sciences, Osaka City University (Japan)

Experimental method:

Different concentrations of LEM were added to the hepatocyte suspension containing $1 \times 10^6$ cells/ml, and cultured for 6 hours. On the other hand, the culture supernatant of LPS (endotoxin)-simulated or -activated macrophages was isolated, and added to said hepatocyte suspension. The subsequent addition of $^3$H-leucinic acid and determination of protein synthesis of hepatocyte on the basis of $^3$H-leucinic acid uptake into acid-insoluble factions were made as in Experiment 4.

Results:

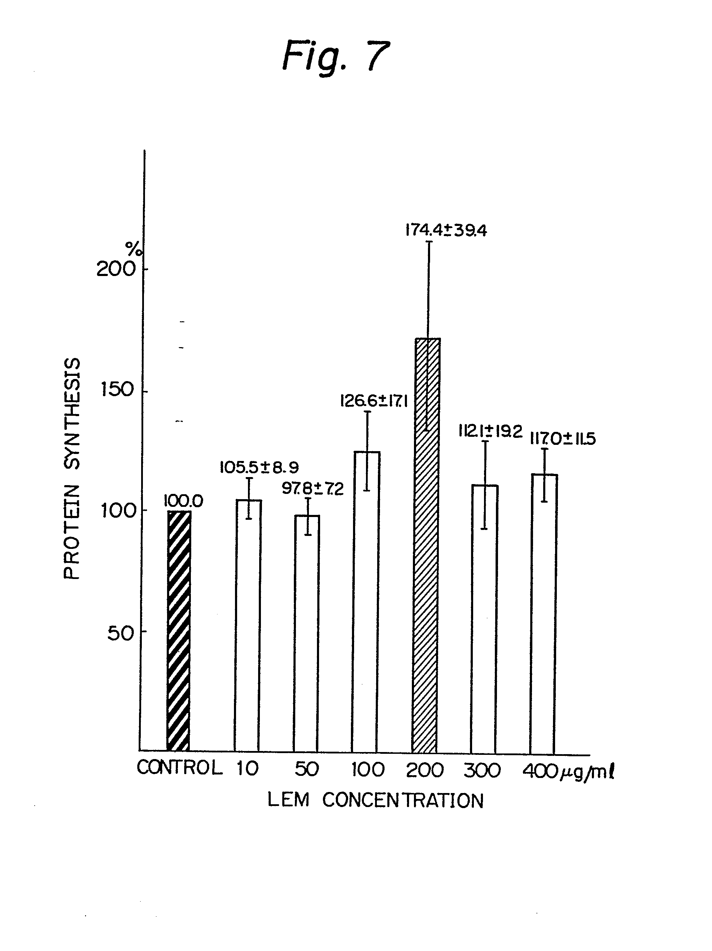

Figure 7 indicates that the protein synthesis of hepatocyte, when the hepatocyte is previously treated with 200 $g^{-6}$/ml of LEM, and is then added to the culture supernatant of activated macrophages, is significantly increased, in comparison with the control in which the hepatocyte is not treated with LEM. It is, therefore, believed that LEM possess the ability to protect the hepatocyte from injury.

Experiment 7:

Object:

This experiment was performed to study the effect of LEM on infectious diseases of eumycetes or fungal infectious diseases under immunosuppression conditions.

Laboratory:

Laboratory of Biological Chemistry, Meiji College of Pharmacy (Japan)

Experimental method:

Cortisone as an immunosuppresive agent was administered to mice ICRK (6 to 7 weeks old), and $1 \times 10^5$/mouse of Candida albicans (J-1012) as challenge eumycetes were then inoculated. Further, before and after inoculation of Candida albicans, and only after inoculation, LEM in the dose of 300 $g^{-6}$/mouse was added to the mice. A vitality percentage of mice was determined. The schedule of grouping used herein is summarized in the following table.

| Group | Cortisone | IEM | Candida albicans | Mouse |
|-------|-----------|-----|------------------|-------|
| I | + | - | + | 5 |
| II | + | + (before and after 2 weeks) | + | 7 |
| III | + | - + (after 2 weeks) | + | 5 |
| IV | - | - | + | 5 |

+: added; and -: not added

Results:

The vitality percentage of mice determined after 25 days of inoculation of Candida albicans is as follows:

Group I 25%, Group II 83%
Group III 100%, and Group IV 50%.

It is clear from this result that LEM effectively protects mice (under immunosuppression conditions) from infectious diseases. Further, the administration of LEM for a long period is not necessary. In fact, it was found that a satisfactory protective effect is attained, even if LEM is administered after infection.

It is believed that the alcohol-purified extract used in Experiment 1 can be used to similar effect in Experiments 2 - 7.

Claims

1.    An immunopotentiation agent comprising two types of peptidoglycans whose peptide portions contain acidic amino acids and whose polysaccharide portions contain arabinose, glucose, galactose, mannose and xylose, the first of which peptidoglycans has a molecular weight of 500,000 to 800,000 and the second of which has a molecular weight of 10,000 to 50,000 and has a higher acidic amino acid content than the first; said agent being obtainable by culturing basidiomycetes mycelia on a xylose-rich solid medium, and extracting the mixture of mycelia and nutrient medium with water.

2.    An agent according to claim 1 which is derived from leninus edodes.

3.    An agent according to claim 1 or claim 2 which is obtainable by being precipitated with an alcohol from said aqueous extract or from an aqueous solution of a dried product initially obtained from said aqueous extract.

4.    A peptidoglycan having a molecular weight of 500,000 to 800,000 as defined in claim 1, when separated from said second peptidoglycan.

5.    A peptidoglycan having a molecular weight of 10,000 to 50,000 as defined in claim 1, when separated from said first peptidoglycan.

6.    A method of preparing an agent according to claim 1 which comprises culturing basidiomycetes mycelia on a xylose-rich solid medium and extracting the mixture of mycelia and nutrient medium with water.

7.    A method according to claim 6 in which the agent is precipitated from the aqueous extract (or from an aqueous solution of a dried product initially obtained from the aqueous extract) by treatment with an alcohol.

8.    A method according to claim 7 wherein the alcohol is ethanol.

9.    A method according to any claims 6 to 8 in which the extraction with water is effected at 40 - 60°C.

10.    A method according to any of claims 6 to 9 in which the extraction with water is effected at a pH of 3 - 8.

11.    A method according to any of claims 6 to 10 which includes the subsequent step of separating the two peptidoglycan fractions of said agent.

# Fig. 1

# Fig. 2

Fig. 3

4/7

0154066

Fig. 4

Fig. 5

# Fig. 6

# Fig. 7

**European Patent Office**

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 088 151 (NODA SHOKUKIN KOGYO CO., LTD.) * Claims 1-4; page 5, line 1 - page 11, line 5; page 19, line 22 - page 21, line 5 * | 1-11 | C 12 P 21/00<br>C 12 P 21/02<br>A 61 K 35/84<br>A 61 K 37/00 //<br>A 61 K 39/39<br>A 61 K 45/05 |
| X | PATENTS ABSTRACTS OF JAPAN, vol. 7, no. 219(C-188)(1364), 29th September 1983; & JP - A - 58 118 519 (NODA SHIYOTSUKIN KOGYO K.K.) 14-07-1983 * Abstract * | 1,2 | |
| D,X | PATENTS ABSTRACTS OF JAPAN, vol. 7, no. 56(C-155)(1201), 8th March 1983; & JP - A - 57 206 618 (NODA SHIYOTSUKIN KOGYO K.K.) 18-12-1982 * Abstract * | 1,2,6 | |
| A | BIOLOGICAL ABSTRACTS, vol. 65, February 1978, no. 17273, Philadelphia, USA; K. TOKIMOTO et al.: "Nutritional aspects of bed-logs of Lentinus edodes (Berk.) Sing. during fruit-body development" & REP TOTTORI MYCOL INST. 15. 65-69, 1977 * Abstract * | 1,2 | TECHNICAL FIELDS SEARCHED (Int. Cl.4)<br><br>C 12 P<br>C 12 D<br>A 61 K |
| | --- -/- | | |

The present search report has been drawn up for all claims

| Place of search | Date of completion of the search | Examiner |
|---|---|---|
| THE HAGUE | 09-11-1984 | DEKEIREL M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

0154066

European Patent Office

EUROPEAN SEARCH REPORT

Application number

EP 84 30 1466

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 93, no. 25, 22nd December 1980, page 484, no. 235182v, Columbus, Ohio, USA;D.W. PARK et al.: "Studies on the constituents of the higher fungi of Korea. XVII. Production of antineoplastic components by the submerged culture of Lentinus edodes" & SOUL TAEHAKKYO YAKHAK NONMUNJI P 1979, 4, 19-30 * Abstract * | 1,2 | |
| A | CHEMICAL AND PHARMACEUTICAL BULLETIN, vol. 30, no. 4, April 1982, pages 1134-1140, Tokyo, JP; M. TOGAMI et al.: "Studies on basidiomycetes. I. Antitumor polysaccharide from bagasse medium on which mycelia of Lentinus edodes (Berk.) Sing. had been grown" * Page 1134, "abstract"; page 1137, figures 3,4 and the last paragraph but one * | 1,2 | TECHNICAL FIELDS SEARCHED (Int. Cl.4) |
| A | CHEMICAL ABSTRACTS, vol. 93, no. 7, 18th August 1980, page 55, no. 61235x, Columbus, Ohio, USA; S. MATSUBARA et al.: "The induction of viral inhibitor(s) in mice treated with biological and synthetic immunopotentiators" & MICROBIOL. IMMUNOL. 1980, 24(1), 87-90 * Abstract * | 1 | |

The present search report has been drawn up for all claims

| Place of search | Date of completion of the search | Examiner |
|---|---|---|
| THE HAGUE | 09-11-1984 | DEKEIREL M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82